# EUROPEAN PATENT SPECIFICATION

(11) **EP 1 038 970 B1**
(45) Date of publication and mention of the grant of the patent: **29.11.2006**
(21) Application number: 00105968.2
(22) Date of filing: 24.03.2000
(51) Int. Cl.: C12P 13/14, C12N 15/31, C07K 14/345, C12R 1/13

(54) **Method for producing L-glutamic acid**
Verfahren zur Herstellung von L-Glutaminsäure
Procédé de préparation de l'acide l-glutamique

(30) Priority: 25.03.1999 JP 8169399
(43) Date of publication of application: 27.09.2000
(73) Proprietor: Ajinomoto Co., Inc., Tokyo 104 (JP)
(72) Inventor: Kimura, Eiichiro, Ajinomoto Co., Inc., Kawasaki-ku, Kawasaki-shi, Kanagawa (JP); Nakamatsu, Tsuyoshi, Ajinomoto Co., Inc., Kawasaki-ku, Kawasaki-shi, Kanagawa (JP); Kurahashi, Osamu, Ajinomoto Co., Inc., Kawasaki-ku, Kawasaki-shi, Kanagawa (JP); Ohtaki, Hiromi, Ajinomoto Co., Inc., Kawasaki-ku, Kawasaki-shi, Kanagawa (JP)
(74) Representative: HOFFMANN EITLE

(56) References cited:
- DE-A- 19 523 279
- KRONEMEYER W ET AL: "STRUCTURE OF THE GLUABCD CLUSTER ENCODING THE GLUTAMATE UPTAKE SYSTEM OF CORYNEBACTERIUM GLUTAMICUM" JOURNAL OF BACTERIOLOGY, WASHINGTON, DC, US, vol. 177, no. 5, March 1995 (1995-03), pages 1152-1158, XP000960684 ISSN: 0021-9193
- BURKOVSKI ANDREAS ET AL: "Characterization of a secondary uptake system for L-glutamate in Corynebacterium glutamicum." FEMS MICROBIOLOGY LETTERS, vol. 136, no. 2, 1996, pages 169-173, XP001029004 ISSN: 0378-1097
- EGGELING L ET AL: "TRANSPORT MUTANTS AND TRANSPORT GENES OF CORYNEBACTERIUM GLUTAMICUM" ANNALS OF THE NEW YORK ACADEMY OF SCIENCES, NEW YORK ACADEMY OF SCIENCES, NEW YORK, NY, US, vol. 782, 15 May 1996 (1996-05-15), pages 191-201, XP000675250 ISSN: 0077-8923
- IKEDA MASATO ET AL: "Tryptophan production by transport mutants of Corynebacterium glutamicum." BIOSCIENCE BIOTECHNOLOGY AND BIOCHEMISTRY, vol. 59, no. 8, 1995, pages 1600-1602, XP001021264 ISSN: 0916-8451
- TAUCH A ET AL: "Isoleucine uptake in Corynebacterium glutamicum ATCC 13032 is directed by the brnQ gene product" ARCHIVES OF MICROBIOLOGY, (APR 1998) VOL. 169, NO. 4, PP. 303-312. PUBLISHER: SPRINGER VERLAG, 175 FIFTH AVE, NEW YORK, NY 10010. ISSN: 0302-8933., XP002180294
- OKAMOTO K ET AL: "Hyperproduction of L- threonine by an Escherichia coli mutant with impaired L- threonine uptake" BIOSCIENCE BIOTECHNOLOGY AND BIOCHEMISTRY, (NOV 1997) VOL. 61, NO. 11, PP 1877-1882. PUBLISHER: JAPAN SOC BIOSCI BIOTECHN AGROCHEM, JAPAN ACAD SOC CTR BLDG, 2-4-6 YAYOI BUNKYO-KU, TOKYO 113, JAPAN. ISSN: 0916-8451., XP001018682
- IKEDA M ET AL: "TRANSPORT OF AROMATIC AMINO ACIDS AND ITS INFLUENCE ON OVERPRODUCTION OF THE AMINO ACIDS IN CORYNEBACTERIUM GLUTAMICUM" JOURNAL OF FERMENTATION AND BIOENGINEERING, SOCIETY OF FERMENTATION TECHNOLOGY, JP, vol. 78, no. 6, 1994, pages 420-425, XP000608032 ISSN: 0922-338X
- KRAMER R: "Genetic and physiological approaches for the production of amino acids" JOURNAL OF BIOTECHNOLOGY, ELSEVIER SCIENCE PUBLISHERS, AMSTERDAM, NL, vol. 45, no. 1, 12 February 1996 (1996-02-12), pages 1-21, XP004036833 ISSN: 0168-1656
- KRAEMER R ET AL: "MECHANISM AND ENERGETICS OF AMINO-ACID TRANSPORT IN CORYNEFORM BACTERIA" BIOCHIMICA ET BIOPHYSICA ACTA, AMSTERDAM, NL, vol. 1187, no. 2, 1994, pages 245-249, XP001028982 ISSN: 0006-3002
- CLEMENT Y ET AL: "IS GLUTAMATE EXCRETED BY ITS UPTAKE SYSTEM IN CORYNEBACTERIUM-GLUTAMICUM? A WORKING HYPOTHESIS" JOURNAL OF GENERAL MICROBIOLOGY, vol. 130, no. 10, 1984, pages 2589-2594, XP001037795 ISSN: 0022-1287

### SEQUENCE LISTING

<110> Ajinomoto Co., Inc.
<120> Method for Producing L-Glutamic Acid
<130> 0P948
<141> 2000-03-24
<150> JP 11-81693
   <151> 1999-03-25
<160> 7
<170> PatentIn Ver. 2.0
<210> 1
   <211> 25
   <212> DNA
   <213> Artificial Sequence
<220>
   <223> Description of Artificial Sequence:primer for PCR
   gactggcagg atcctgatta taagg 25
<400> 2
<210> 1
   <211> 30
   <212> DNA
   <213> Artificial Sequence
<220>
   <223> Description of Artificial Sequence:primer for PCR
<400> 2
   cgcgtctaga ggcgcttgag caaatcgacc 30
<210> 3
   <211> 30
   <212> DNA
   <213> Artificial Sequence
<220>
   <223> Description of Artificial Sequence:primer for PCR
<400> 3
   aggtgaattc cggacaggat cggagactac 30
<210> 4
   <211> 25
   <212> DNA
   <213> Artificial Sequence
<220>
   <223> Description of Artificial Sequence:primer for PCR
<400> 4
   ttgacttgcc ggatccggat ggtcc 25
<210> 5
   <211> 30
   <212> DNA
   <213> Artificial Sequence
<220>
   <223> Description of Artificial Sequence:primer for PCR
<400> 5
   tcatgaattc ctcaccgttt tgaatgaggg 30
<210> 6
   <211> 30
   <212> DNA
   <213> Artificial Sequence
<220>
   <223> Description of Artificial Sequence:primer for PCR
<400> 6
   gatgagatct cgttccaaca ggctcatcgc 30
<210> 7
   <211> 30
   <212> DNA
   <213> Artificial Sequence
<220>
   <223> Description of Artificial Sequence:primer for PCR
<400> 7
   aagcagatct tcgtgtgttg ttcatggcgg 30

### SEQUENCE LISTING

<110> Ajinomoto Co., Inc.
<120> Method for Producing L-Glutamic Acid
<130> OP948
<141> 2000-03-24
<150> JP 11-81693
   <151> 1999-03-25
<160> 7
<170> PatentIn Ver. 2.0
<210> 1
   <211> 25
   <212> DNA
   <213> Artificial Sequence
<220>
   <223> Description of Artificial Sequence:primer for PCR
   gactggcagg atcctgatta taagg 25
<400> 2
   <210> 1
   <211> 30
   <212> DNA
   <213> Artificial Sequence
<220>
   <223> Description of Artificial Sequence:primer for PCR
<400> 2
   cgcgtctaga ggcgcttgag caaatcgacc 30
<210> 3
   <211> 30
   <212> DNA
   <213> Artificial Sequence
<220>
   <223> Description of Artificial Sequence:primer for PCR
<400> 3
   aggtgaattc cggacaggat cggagactac 30
<210> 4
   <211> 25
   <212> DNA
   <213> Artificial Sequence
<220>
   <223> Description of Artificial Sequence:primer for PCR
<400> 4
   ttgacttgcc ggatccggat ggtcc 25
<210> 5
   <211> 30
   <212> DNA
   <213> Artificial Sequence
<220>
   <223> Description of Artificial Sequence:primer for PCR
<400> 5
   tcatgaattc ctcaccgttt tgaatgaggg 30
<210> 6
   <211> 30
   <212> DNA
   <213> Artificial Sequence
<220>
   <223> Description of Artificial Sequence:primer for PCR
<400> 6
   gatgagatct cgttccaaca ggctcatcgc 30
<210> 7
   <211> 30
   <212> DNA
   <213> Artificial Sequence
<220>
   <223> Description of Artificial Sequence:primer for PCR
<400> 7
   aagcagatct tcgtgtgttg ttcatggcgg 30

## Description

### BACKGROUND OF THE TNVENTTON

### Technical Field

The present invention relates to an L-glutamic acid-producing bacterium and a method for producing L-glutamic acid by fermentation utilizing the bacterium. L-glutamic acid is an amino acid important for foodstuffs, medical supplies and so forth.

### Related Art

Heretofore, L-glutamic acid has been produced by a fermentation method using coryneform L-glutamic acid-producing bacteria belonging to the genus *Brevibacterium*, or *Corynebacterium* (Amino Acid Fermentation, Gakkai Shuppan Center, pp.195-215, 1986). As such coryneform bacteria, wild strains isolated from nature or mutants thereof are used to improve the productivity.

Moreover, there have also been disclosed various techniques for promoting L-glutamic acid-producing ability by enhancing genes for enzymes involved in the L-glutamic acid biosynthetic system through recombinant DNA techniques. For example, Japanese Patent Laid-open Publication No. 63-214189 discloses a technique for elevating the L-glutamic acid-producing ability by enhancing a glutamate dehydrogenase gene, isocitrate dehydrogenase gene, aconitate hydratase gene, and citrate synthase gene.

On the other hand, as for L-threonine, there has been known a technique of disruption of an uptake system of the amino acid in order to increase the production of the amino acid (Okamoto, K. *et al*., *Biosci. Biotech*. *Biochem*., *61* (11), 1877-1882 (1997)).

For L-glutamic acid, the structure of the gene-cluster of the uptake system for L-glutamic acid (*gluABCD* operon) in *Corynebacterium glutamicum* has been known (Kronemeyer, W. *et al*., *J*. *Bacteriol*., *177* (5), 1152-1158 (1995)). Moreover, Kronemeyer *et al*. produced a strain in which the L-glutamic acid uptake system encoded by the *gluABCD* operon was deleted, and studied about the excretion of L-glutamic acid using this strain. In this study, they concluded that the excretion of L-glutamic acid of *Corynebacterium glutamicum* did not depend on *gluABCD*, and depended on other uptake and excretion mechanisms. Furthermore, an uptake system of L-glutamic acid other than that encoded by *gluABCD* has also been reported (Burkovski, A. *et al*., *FEMS Microbiology Letters*, *136*, 169-173 (1996)). These reports suggest that the accumulation amount of L-glutamic acid in the medium is not affected even if at least the L-glutamic acid uptake system encoded by the *gluABCD* operon is deleted. Therefore, it has not been attempted to improve the L-glutamic acid-producing ability by disruption of the uptake system of L-glutamic acid encoded by *gluABCD*.

### DETAILED DESCRIPTION OF THE INVENTION

The object of the present invention is to breed a bacterial strain having high L-glutamic acid-producing ability, and thereby provide a method for more efficiently producing L-glutamic acid at low cost in order to respond to further increase of the demand of L-glutamic acid.

In order to achieve the aforementioned object, the inventors of the present invention studied assiduously. As a result, they found that an L-glutamic acid-producing bacterium of coryneform bacteria whose *gluABCD* operon was deleted had high L-glutamic acid-producing ability contrary to the suggestion by the aforementioned prior art, and thus accomplished the present invention.

That is, the present invention provides the followings.
(1) A method for producing L-glutamic acid, comprising the steps of culturing a coryneform bacterium which has L-glutamic acid-producing ability in a medium to produce and accumulate L-glutamic acid in the medium, and collecting the L-glutamic acid from the medium, wherein an L-glutamic acid uptake system is deleted or decreased in the coryneform bacterium.
(2) The method according to (1), wherein the L-glutamic acid uptake system is encoded by the *gluABCD* operon.
(3) The method according to (2), wherein at least one of expression products of the *gluABCD* operon is deleted in the coryneform bacterium.
(4) The method according to (3), wherein at least *gluA* is deleted in the coryneform bacterium.
(5) The method according to (4), wherein all of *gluA*, *gluB*, *gluC* and *gluD* are deleted in the coryneform bacterium.

According to the present invention, L-glutamic acid can be produced in a medium which contains high concentration of L-glutamic acid at a higher yield compared with conventional techniques.

For the present invention, the term "L-glutamic acid-producing ability" refers to an ability of a coryneform bacterium used for the present invention to accumulate L-glutamic acid in a medium when the bacterium is cultured in the medium.

Hereafter, the present invention will be explained in detail.

The coryneform bacterium used for the present invention is a coryneform bacterium having L-glutamic acid-producing ability, in which an L-glutamic acid uptake system is deleted or decreased.

Bacteria belonging to the genus *Corynebacterium* as referred to herein are a group of microorganisms defined in Bergey's Manual of Determinative Bacteriology, 8th Ed., p. 599 (1974). The bacteria are aerobic, Grampositive, nonacid-fast bacilli not having the ability to sporulate, and include bacteria which had been classified as bacteria belonging to the genus *Brevibacterium* but have now been unified into the genus *Corynebacterium* [see Int. *J*. *Syst*. *Bacteriol*., *41*, 255 (1981)] and also include bacteria of the genus *Brevibacterium* and Microbacterium which are closely related to the genus *Corynebacterium*. Examples of coryneform bacteria preferrably used for producing L-glutamic acid include the followings.
*Corynebacterium acetoacidophilum*
*Corynebacterium acetoglutamicum*
*Corynebacterium alkanolyticum*
*Corynebacterium callunae*
*Corynebacterium glutamicum*
*Corynebacterium lilium (Corynebacterium glutamicum*)
*Corynebacterium melassecola*
*Corynebacterium thermoaminogenes*
*Corynebacterium herculis*
*Brevibacterium divaricatum* (*Corynebacterium glutamicum*)
*Brevibacterium flavum* (*Corynebacterium glutamicum*)
*Brevibacterium immariophilum*
*Brevibacterium lactofermentum* (*Corynebacterium glutamicum*)
*Brevibacterium roseum*
*Brevibacterium saccharolyticum*
*Brevibacterium thiogenitalis*
*Brevibacterium ammoniagenes* (*Corynebacterium ammoniagenes*)
*Brevibacterium album*
*Brevibacterium cerinum*
*Microbacterium ammoniaphilum*

Specifically, the following strains of these bacteria are exemplified:
*Corynebacterium acetoacidophilum* ATCC13870
*Corynebacterium acetoglutamicum* ATCC15806
*Corynebacterium alkanolyticum* ATCC21511
*Corynebacterium callunae* ATCC15991
*Corynebacterium glutamicum* ATCC13020, 13032, 13060
*Corynebacterium lilium* (*Corynebacterium glutamicum*) ATCC15990
*Corynebacterium melassecola* ATCC17965
*Corynebacterium thermoaminogenes* AJ12340 FERM BP-1539)
*Corynebacterium herculis* ATCC13868
*Brevibacterium divaricatum* (*Corynebacterium glutamicum*) ATCC14020
*Brevibacterium flavum* (*Corynebacterium glutamicum*) ATCC13826, ATCC14067
*Brevibacterium immariophilum* ATCC14068
*Brevibacterium lactofermentum (Corynebacterium glutamicum*) ATCC13665, ATCC13869
*Brevibacterium roseum* ATCC13825
*Brevibacterium saccharolyticum* ATCC14066
*Brevibacterium thiogenitalis* ATCC19240
*Corynebacterium ammoniagenes* (*Brevibacterium* ammoniagenes) ATCC6871
*Brevibacterium album* ATCC15111
*Brevibacterium cerinum* ATCC15112
*Microbacterium ammoniaphilum* ATCC15354

The aforementioned deletion or decrease of an L-glutamic acid uptake system of coryneform bacteria can be attained by mutating or disrupting a gene coding for the uptake system using a mutagenesis treatment or a genetic recombination technique. The term " L-glutamic acid uptake system is deleted or decreased" means to make the uptake system not function normally, and it includes that at least one of proteins constituting the uptake system is deleted or the activity of the protein is decreased, and that two or more of the proteins are deleted or the activities of the proteins are decreased. Further, the term "a protein is deleted" used herein include both of a case where the protein is not expressed at all, and a case where a protein that does not normally function is expressed.

The gene coding for the L-glutamic acid uptake system can be disrupted by gene substitution utilizing homologous recombination. A gene on a chromosome of a coryneform bacterium can be disrupted by transforming the coryneform bacterium with DNA containing a gene modified by deleting its internal sequence (deletion type gene) so that the uptake system should not function normally to cause recombination between the deletion type gene and a normal gene on the chromosome. Such gene disruption utilizing homologous recombination has already been established, and there have been known methods therefor utilizing linear DNA, plasmid containing a temperature sensitive replication origin and so forth. A method utilizing a plasmid containing a temperature sensitive replication origin is preferred.

As a gene coding for an L-glutamic acid uptake system, the *gluABCD* operon has been known (Kronemeyer, W. *et al*., *J*. *Bacteriol*., *177* (5), 1152-1158 (1995)). Moreover, an L-glutamic acid uptake system other than that encoded by the *gluABCD* operon has also been known (Burkovski, A. *et al*., *FEMS Microbiology Letters*, *136*, 169-173 (1996)). Although any of such genes may be disrupted, it is preferred that the uptake system encoded by the *gluABCD* operon is disrupted.

Because the nucleotide sequence of this operon has been known (GenBank/EMBL/DDBJ Accession X81191), this operon or each structural gene in the *gluABCD* operon can be isolated from chromosome DNA of coryneform bacteria by PCR using primers produced based on the nucleotide sequence. A certain region can be excised from the thus obtained gene fragment with one or more restriction enzymes, and at least a part of a coding region or an expression control sequence such as promoter can be deleted to prepare a deletion type gene.

Further, a deletion type gene can also be obtained by performing PCR using primers designed so that a part of a gene should be deleted. For example, by using the primers having the nucleotide sequences shown as SEQ ID NOS: 1 and 2 in Sequence Listing, a *gluD* gene having a deletion of a part of 5' sequence can be obtained. Further, by using the primers having the nucleotide sequences shown as SEQ ID NOS: 3 and 4, a *gluA* gene having a deletion of a part of 3' sequence can be obtained. When gene substitution is performed by using the deletion type *gluA* gene or *gluD* gene obtained by using those primers, the *gluA* gene or the *gluD* gene can be disrupted. Further, if these deletion type *gluA* gene and deletion type *gluD* gene are ligated, and the obtained fusion gene is used for gene substitution, all of *gluA*, *gluB*, *gluC* and *gluD* can be disrupted. Furthermore, when PCR is performed by using a plasmid containing a *gluA* gene that has been obtained by using primers having the nucleotide sequences shown as SEQ ID NOS: 3 and 5 as a template, and primers having the nucleotide sequences shown as SEQ ID NOS: 6 and 7, and the amplification product is cyclized, there can be obtained a plasmid containing *gluA* gene including a deletion of an internal sequence and having 5' region and 3' region ligated in-flame. When gene substitution is performed by using this plasmid, only the *gluA* gene can be disrupted.

Moreover, primers other than those exemplified above can also be designed by the methods well known to those skilled in the art, and an arbitrary structural gene in the *gluABCD* operon can be disrupted by using such primers. Alternatively, the uptake system can be deleted by deleting an expression control sequence of the *gluABCD* operon such as a promoter so that the gene cannot be expressed.

While the gene substitution of the *gluABCD* gene-cluster (henceforth referred to simply as "*gluABCD* gene") will be explained below, an arbitrary structural gene or expression control sequence can be similarly deleted.

The *gluABCD* gene on a host chromosome can be replaced with a deletion type *gluABCD* gene as follows. That is, a recombinant DNA is constructed by insertion of a temperature sensitive replication origin, a deletion type *gluABCD* gene and a maker gene expressing resistance to a drug such as chloramphenicol, tetracycline and streptomycin, and a coryneform bacterium is transformed with this recombinant DNA. Then, the transformant strain can be cultured at a temperature at which the temperature sensitive replication origin does not function, and then cultured in a medium containing a corresponding drug to obtain a transformant strain in which the recombinant DNA is integrated into chromosomal DNA.

In such a strain in which a recombinant DNA is integrated into a chromosome as described above, recombination of the *gluABCD* gene sequence originally present on the chromosome has been caused, and two fusion genes of the chromosomal *gluABCD* gene and the deletion type *gluABCD* gene are inserted into the chromosome, between which the other parts of the recombinant DNA (the vector protion, temperature sensitive replication origin, and drug resistance marker) are present. Therefore, because the normal *gluABCD* gene is dominant in this state, the transformant strain expresses the L-glutamic acid uptake system.

Then, in order to leave only the deletion type *gluABCD* gene on the chromosome DNA, one copy of the *gluABCD* gene is eliminated from the chromosome DNA together with the vector protion (including temperature sensitive replication origin and drug resistance marker) by recombination of the two *gluABCD* gene. Upon the recombination, the normal *gluABCD* gene may be left on the chromosome DNA and the deletion type *gluABCD* gene may be excised, or the deletion type *gluABCD* gene may be left on the chromosome DNA and the normal *gluABCD* gene may be excised. In both of the cases, the excised DNA is retained on the plasmid in a cell when the cell is cultured at a temperature at which the temperature sensitive replication origin functions. Such DNA on the plasmid is eliminated from the cell when the cell is cultured at a temperature at which the temperature sensitive replication origin does not function. It can be confirmed which genes are left on the chromosome DNA by investigating the structure of the *gluABCD* gene on the chromosome by PCR, hybridization or the like.

When such a *gluABCD* gene-disrupted strain produced as described above is cultured at a temperature at which the temperature sensitive replication origin functions (for example, low temperature), the *gluABCD* gene will be retained in its cell. When it is cultured at a temperature at which the temperature sensitive replication origin does not function (for example, elevated temperature), the *gluABCD* gene will be eliminated from the cell.

Examples of the plasmid which has a temperature sensitive replication origin functioning in coryneform bacterial cells include, pHS4, pHSC4, pHS22, pHSC22, pHS23, pHSC23 (as for these, see Japanese Patent Publication (Kokoku) No. 7-108228) and so forth. These temperature sensitive plasmids can autonomously replicate at a temperature of about 10-32°C, but cannot autonomously replicate at a temperature of about 34°C or higher in a coryneform bacterial cell.

After the *gluABCD* gene on the chromosome are disrupted as described above, the genes-disrupted strain is preferably introduced with *recA*^{*-*} because such introduction of *recA*⁻ prevents the *gluABCD* gene on the plasmid from being integrated again into the chromosome again during culture at a low temperature.

The coryneform bacterium used for the present invention may have enhanced activity of an enzyme for catalyzing the biosynthesis of L-glutamic acid in addition to the deletion or decrease of L-glutamic acid uptake system. Illustrative examples of the enzyme for catalyzing the biosynthesis of L-glutamic acid include glutamate dehydrogenase, glutamine synthetase, glutamate synthase, isocitrate dehydrogenase, aconitate hydratase, citrate synthase, pyruvate carboxylase, phosphoenolpyruvate carboxylase, phosphoenolpyruvate synthase, enolase, phosphoglyceromutase, phosphoglycerate kinase, glyceraldehyde-3-phosphate dehydrogenase, triosephosphate isomerase, fructose bisphosphate aldolase, phosphofructokinase, glucose phosphate isomerase and the like.

Further, in the coryneform bacterium used for the present invention, an enzyme that catalyzes a reaction for generating a compound other than L-glutamic acid by branching off from the biosynthetic pathway of L-glutamic acid may be decreased of or deleted. Illustrative examples of the enzyme which catalyzes a reaction for generating a compound other than L-glutamic acid by branching off from the biosynthetic pathway of L-glutamic acid include a-ketoglutarate dehydrogenase, isocitrate lyase, phosphate acetyltransferase, acetate kinase, acetohydroximate synthase, acetolactate synthase, formate acetyltransferase, lactate dehydrogenase, glutamate decarboxylase, 1-pyrroline dehydrogenase and the like.

Furthermore, by introducing a thermosensitive mutation for a biotin activity inhibiting substance such as surface active agents into a coryneform bacterium having L-glutamic acid-producing ability, the bacterium becomes to be able to produce L-glutamic acid in a medium containing an excessive amount of biotin in the absence of a biotin activity inhibiting substance (see WO96/06180). As such a coryneform bacterium, the Brevibacterium lactofermentum AJ13029 strain disclosed in WO96/06180 can be mentioned. The AJ13029 strain was deposited at the National Institute of Bioscience and Human-Technology, Agency of Industrial Science and Technology on September 2, 1994, and received an accession number of FERM P-14501. Then, its was transferred to an international deposition under the provisions of the Budapest Treaty on August 1, 1995, and received an accession number of FERM BP-5189.

When a coryneform bacterium having L-glutamic acid-producing ability, in which the L-glutamic acid uptake system is deleted or decreased, is cultured in a suitable medium, L-glutamic acid is accumulated in the medium. Because of the deletion or decrease of L-glutamic acid uptake system in the coryneform bacterium used for the present invention, L-glutamic acid secreted from the cell is prevented from being taken up again into the cell. As a result, the accumulation amount of L-glutamic acid in the medium is increased. According to the method of the present invention, improvement of interval yield (ratio of the accumulation amount of L-glutamic acid to the consumption of saccharides in a certain period of cultivation) can be expected when L-glutamic acid concentration in the medium becomes high. In particular, when a highly productive strain that shows a high L-glutamic acid concentration in a medium during fermentation is used, a marked effect can be obtained.

The medium used for producing L-glutamic acid by utilizing the microorganism of the present invention is a usual medium that contains a carbon source, a nitrogen source, inorganic ions and other organic trace nutrients as required. As the carbon source, it is possible to use sugars such as glucose, lactose, galactose, fructose, starch hydrolysate and molasses or the like; alcohols such as ethanol, inositol; or organic acids such as acetic acid, fumaric acid, citric acid and succinic acid or the like.

As the nitrogen source, there can be used inorganic ammonium salts such as ammonium sulfate, ammonium nitrate, ammonium chloride, ammonium phosphate and ammonium acetate, ammonia, organic nitrogen such as peptone, meat extract, yeast extract, corn steep liquor and soybean hydrolysates, ammonia gas, aqueous ammonia and so forth.

As the inorganic ions (or sources thereof), added is a small amount of potassium phosphate, magnesium sulfate, iron ions, manganese ions and so forth. As for the organic trace nutrients, it is desirable to add required substances such as vitamin B1, yeast extract and so forth in a suitable amount as required.

The culture is preferably performed under an aerobic condition attained by shaking, stirring for aeration or the like for 16 to 72 hours. The culture temperature is controlled to be at 30°C to 45°C, and pH is controlled to be 5 to 9 during the culture. For such adjustment of pH, inorganic or organic acidic or alkaline substances, ammonia gas and so forth can be used.

Collection of L-glutamic acid from fermentation broth can be attained by, for example, methods utilizing ion exchange resin, crystallization and so forth. Specifically, L-glutamic acid can be adsorbed or isolated by an anion exchange resin, or crystallized by neutralization.

### BRIEF DESCRIPTION OF THE DRAWINGS

Fig. 1 shows the scheme of construction of a plasmid pTΔAD for disrupting *gluABCD* gene.
Fig. 2 shows the scheme of construction of a plasmid pTΔA for disruption of *gluA*.

### BEST MODE FOR CARRYING OUT THE INVENTION

The present invention will be further specifically explained hereinafter with reference to the following examples.

### <1> Construction of plasmid for disruption of gluABCD gene

In order to create a *gluABCD* gene-disrupted strain of coryneform bacterium by homologous recombination using a temperature sensitive plasmid, a plasmid for disruption of the *gluABCD* gene was constructed.

First, a deletion type *gluABCD* gene was constructed by cloning a *gluD* gene having a deletion of 5' sequence, and ligating it with a *gluA* gene having a deletion of 3' sequence. Specifically, a fragment of about 300 bp from a *Bam*HI site present in *gluD* to a site about 270 bp downstream from *gluD* was amplified from chromosomal DNA of *Brevibacterium lactofermentum* ATCC13869, which was a wild-type strain of coryneform bacterium, by PCR utilizing oligonucleotides having the nucleotide sequences represented as SEQ ID NOS: 1 and 2 as primers. This amplified fragment was digested with *Bam*HI and *Xba*I, and the obtained fragment was ligated to pHSG299 (produced by Takara Shuzo) digested with *Bam*HI and *Xba*I using T4 ligase (produced by Takara Shuzo) to obtain a plasmid pHSGΔgluD.

Then, a fragment of about 300 bp from a site about 180 bp upstream from *gluA* to the *Bam*HI site present in *gluA* was amplified from chromosomal DNA of *Brevibacterium lactofermentum* ATCC13869 by PCR utilizing oligonucleotides having the nucleotide sequences represented as SEQ ID NOS: 3 and 4 as primers. This amplified fragment was digested with *Eco*RI and *Bam*HI, and the obtained fragment was ligated to pHSGΔgluD digested with *Eco*RI and *Bam*HI by using T4 ligase to obtain a plasmid pHSGΔgluAD. This plasmid had a structure in which *gluB* and *gluC* were deleted, and parts of *gluA* and *gluD* were ligated.

Then, in order to make pHSGΔgluAD autonomously replicable in coryneform bacteria, a temperature sensitive replication origin derived form a plasmid autonomously replicable in coryneform bacteria was introduced into the unique *Hin*cII cleavage site in pHSGΔgluAD. Specifically, the following procedure was used.

A plasmid pHSC4 containing a temperature sensitive replication origin (see Japanese Patent Laid-open Publication (Kokai) No. 5-7491) was digested with *Bam*HI and *Kpn*I. The both termini of the obtained DNA fragment was blunt-ended using Blunting Kit (produced by Takara Shuzo), ligated with a *Kpn*I linker (produced by Takara Shuzo), and then allowed to cause self-ligation to obtain pKCT4. pHSC4 was a plasmid obtained as follows. That is, a DNA fragment containing a replication origin was excised from a plasmid pAJ1844 (see Japanese Patent Laid-open Publication (Kokai) No. 58-216199), which had a replication origin derived from pHM1519 (K. Miwa *et al*., *Agric*. *Biol*. *Chem., 48*, 2901-2903 (1984), Japanese Patent Laid-open Publication (Kokai) No. 58-77895), and ligated to a plasmid for *Escherichia coli*, pHSG298, to obtain a shuttle vector pHK4. This pHK4 was treated with hydroxylamine to obtain a plasmid pHS4 modified to be temperature sensitive. The temperature sensitive replication origin was excised from pHS4, and ligated to pHSG398 to obtain pHSC4. *Escherichia coli* AJ12571 harboring pHSC4 was deposited at the National Institute of Bioscience and Human-Technology, Agency of Industrial Science and Technology on October 11, 1990, and received the accession number of FERM P-11763. Then, it was transferred to international deposit under the Budapest Treaty on August 26, 1991, and received the accession number of FERM BP-3524.

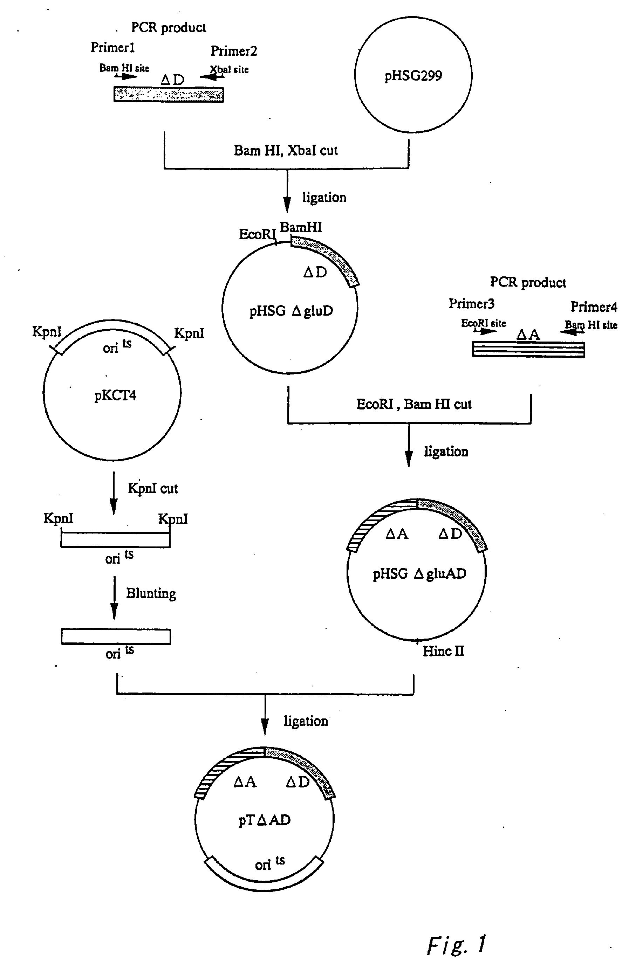

pKCT4 produced as described above had a structure where the replication origin derived from pHM1519 modified to be temperature sensitive was inserted into *Kpn*I site of pHSG399. A fragment containing a temperature sensitive replication origin was obtained by digesting pKCT4 with *Kpn*I, blunt-ended by using Blunting Kit (produced by Takara Shuzo), and ligated to pHSGΔgluAD digested with *Hinc*II to obtain pTΔAD (Fig. 1).

### <2> Construction of plasmid for disruption of gluA gene

In order to create a coryneform bacterium in which only the *gluA* gene was disrupted, a plasmid for disruption of the *gluA* gene was constructed.

A DNA fragment of about 1500 bp containing the *gluA* gene was amplified from chromosomal DNA of *Brevibacterium lactofermentum* ATCC13869 by PCR utilizing oligonucleotides having the nucleotide sequences shown as SEQ ID NOS: 3 and 5 as primers, and the amplified fragment was digested with *Eco*RI, and ligated to pHSG299 (produced by Takara Shuzo) digested with *Eco*RI by using T4 ligase (produced by Takara Shuzo) to obtain a plasmid pHSGgluA.

Then, the 5' region and the 3' region of the *gluA* gene and the vector segment, except for the internal region of the *gluA* gene, were amplified by PCR utilizing oligonucleotides having the nucleotide sequences shown as SEQ ID NOS: 6 and 7 as primers, and pHSGgluA as a template. The aforementioned primers were designed so that it should contain a *Bgl*II recognition sequence. The amplified fragment was digested with *Bgl*II, and allowed to cause self-ligation in the presence of T4 ligase to obtain a plasmid pHSGΔgluA. This plasmid contained deletion of about 250 bp of internal sequence among the about 730 bp open reading frame of *gluA*, and had a structure where the 5' region and the 3' region were ligated in-frame.

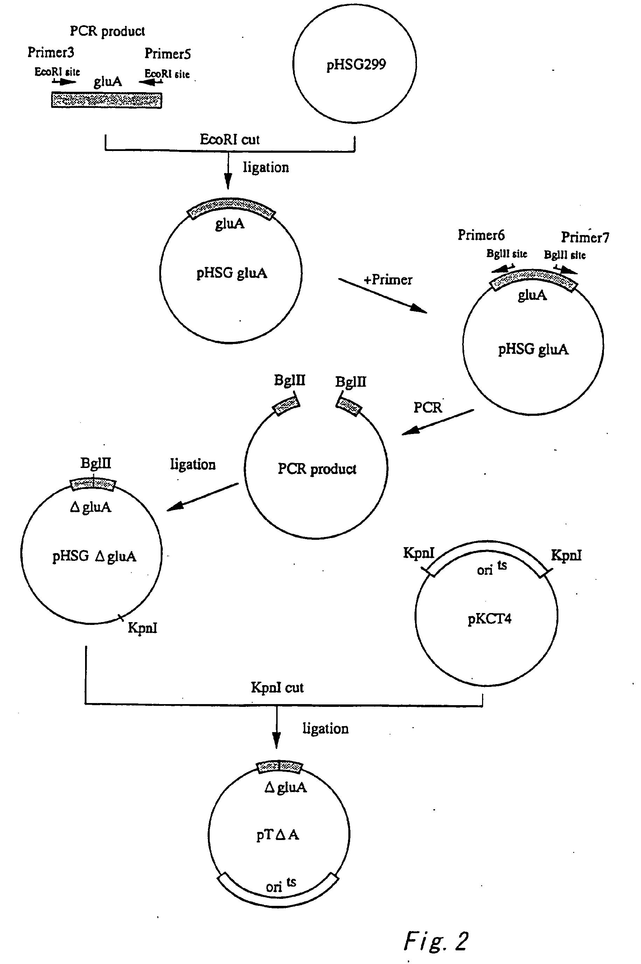

Then, in order to make pHSGΔgluA autonomously replicable in coryneform bacteria, a temperature sensitive replication origin derived from a plasmid autonomously replicable in coryneform bacteria was introduced into the unique *Kpn*I cleavage site in pHSGΔgluA. Specifically, pKCT4 was digested with *Kpn*I to obtain a DNA fragment containing a replication origin, and the obtained fragment was inserted into *Kpn*I site of pHSGΔgluA to obtain pTΔA (Fig. 2).

### <3> Creation of gluABCD gene-disrupted strain and gluA gene-disrupted strain

The plasmids for disrupting genes obtained as described above, pTΔAD and pTΔA, were introduced into a wild-type strain, *Brevibacterium lactofermentum* ATCC13869 strain, by using the electric pulse method to obtain ATCC13869/pTΔAD and ATCC13869/pTΔA. Gene disruption was performed by using these transformant strains.

Specifically, ATCC13869/pTΔAD and ATCC13869/pTΔA were cultured at 25°C in CM2B broth for 24 hours with shaking, and inoculated to CM2B medium containing 25 µg/ml of kanamycin. Strains into which the plasmids were introduced were obtained as strains that formed colonies at 34°C, at which temperature the temperature sensitive replication origin did not function. Then, the strains that became sensitive to kanamycin at 34°C were obtained by the replica method. Chromosome DNA of these sensitive strains was obtained in a conventional manner. The structures of the *gluABCD* gene and the *gluA* gene on the chromosome was examined by PCR and sequencing to confirm that these genes should be replaced with those of the deletion type, and the strains containing the deletion type genes were designated as ΔAD strain and ΔA strain, respectively.

The ΔAD strain and the ΔA strain were given with private numbers of AJ13587 and AJ13588, respectively, and deposited at the National Institute of Bioscience and Human-Technology, Agency of Industrial Science and Technology (postal code: 305-8566, 1-3 Higashi 1-chome, Tsukuba-shi, Ibaraki-ken, Japan) on March 23, 1999, and received accession numbers of FERM P-17327 and FERM P-17328, respectively, and then, transferred from the original deposit to international deposit based on Budapest Treaty on February 14, 2000, and have been deposited as deposit numbers of FERM BP-7028 and FERM BP-7029, respectively.

### <4> Evaluation of L-glutamic acid-producing ability of strains ΔAD and ΔA

Culture of the strains ATCC13869, ΔAD and ΔA for the production of L-glutamic acid was performed as follows. These strains that had been refreshed by culture in a CM2B plate medium were cultured in two kinds of mediums, a medium containing 80 g of glucose, 1 g of KH₂PO₄, 0.4 g of MgSO₄•7H₂O, 30 g of (NH₄)₂SO₄, 0.01 g of FeSO₄•7H₂O, 0.01 g of MnSO₄•7H₂O, 15 ml of soybean hydrolysate solution, 200 µg of thiamin hydrochloride, 3 pg of biotin, and 50 g of CaCO₃ in 1 L of deionized water (prepared at pH 8.0 by using KOH), and a medium further containing 50 g/L of L-glutamic acid in the foregoing medium, at 31.5°C. After the cultivation, the amounts of accumulated L-glutamic acid in the mediums, and absorbance at 620 nm of the mediums diluted 51 times were measured. The results obtained for the medium with no addition of L-glutamic acid were shown in Table 1. The results obtained for the medium added with L-glutamic acid were shown in Table 2.

**Table 1**

| Strain | OD₆₂₀ | L-glutamic acid (g/L) | Yield (%) |
|---|---|---|---|
| ATCC 13869 | 0.937 | 40.8 | 50.4 |
| ΔAD | 1.127 | 36.3 | 44.9 |
| ΔA | 0.766 | 44.3 | 54.8 |

**Table 2**

| Strain | OD₆₂₀ | L-glutamic acid (g/L)* | Yield (%) |
|---|---|---|---|
| ATCC 13869 | 0.845 | 29.5 | 43.9 |
| ΔAD | 0.927 | 30.0 | 44.6 |
| ΔA | 0.749 | 32.0 | 47.6 |

| | | | |
|---|---|---|---|
| * The amounts do not include the L-glutamic acid added to the medium. | | | |

These results show that the accumulation amount and yield of L-glutamic acid were improved for both of the ΔA strain and the ΔAD strain when the medium contained L-glutamic acid at a high concentration. Further, the yield of L-glutamic acid was improved by the ΔA strain even in the medium not containing L-glutamic acid.

## Claims

1. A method for producing L-glutamic acid, comprising the steps of culturing a coryneform bacterium which has L-glutamic acid-producing ability in a medium to produce and accumulate L-glutamic acid in the medium, and collecting the L-glutamic acid from the medium, wherein an L-glutamic acid uptake system is deleted or decreased in the coryneform bacterium, wherein the L-glutamic acid uptake system is encoded by the *gluABCD* operon and wherein at least one of expression products of the *gluABCD* operon is deleted in the coryneform bacterium.

2. The method of claim 1, wherein at least *gluA* is deleted in the coryneform bacterium.

3. The method of claim 2, wherein all of *gluA*, *gluB*, *gluC* and *gluD* are deleted in the coryneform bacterium

## Patentansprüche

1. Verfahren zur Herstellung von L-Glutaminsäure, umfassend die Schritte der Kultivierung eines coryneformen Bakteriums, das eine L-Glutaminsäureerzeugende Fähigkeit hat, in einem Medium, um L-Glutaminsäure in dem Medium zu erzeugen und anzuhäufen, und Sammeln der L-Glutaminsäure aus dem Medium, wobei ein L-Glutaminsäure-Aufnahmesystem in dem coryneformen Bakterium deletiert oder vermindert ist, wobei das L-Glutaminsäure-Aufnahmesystem durch das gluABCD-Operon codiert wird und wobei mindestens eines der Expressionsprodukte des gluABCD-Operons in dem coryneformen Bakterium deletiert ist.

2. Verfahren gemäss Anspruch 1, wobei mindestens gluA in dem coryneformen Bakterium deletiert ist.

3. Verfahren gemäss Anspruch 2, worin alle von gluA, gluB, gluC und gluD in dem coryneformen Bakterium deletiert sind.

## Revendications

1. Procédé pour la préparation de l'acide L-glutamique, comprenant les étapes de mise en culture d'une bactérie coryneforme qui possède une aptitude de production d'acide L-glutamique dans un milieu pour produire et accumuler l'acide L-glutamique dans le milieu, et recueillir l'acide L-glutamique du milieu, dans lequel un système d'absorption d'acide L-glutamique est supprimé ou réduit dans la bactérie coryneforme, dans lequel le système d'absorption de l'acide L-glutamique est codé par l'opéron *glu*ABCD et dans lequel au moins l'un des produits d'expression de l'opéron *glu*ABCD est supprimé dans la bactérie coryneforme.

2. Procédé selon la revendication 1, dans lequel au moins *glu*A est supprimé dans la bactérie coryneforme.

3. Procédé selon la revendication 2, dans lequel la totalité de *glu*A, *glu*B, *glu*C et *glu*D sont supprimés dans la bactérie coryneforme.
